(19) Europäisches Patentamt
European Patent Office
Office européen des brevets

(11) Veröffentlichungsnummer: **0 348 803 A1**

(12) **EUROPÄISCHE PATENTANMELDUNG**

(21) Anmeldenummer: **89111256.7**

(51) Int. Cl.4: **C07D 233/92**

(22) Anmeldetag: **21.06.89**

(30) Priorität: **29.06.88 DE 3821884**

(43) Veröffentlichungstag der Anmeldung: **03.01.90 Patentblatt 90/01**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Köhler, Hermann, Dr.**
**Röntgenstrasse 7**
**D-6711 Beindersheim(DE)**
Erfinder: **Dockner, Toni, Dr.**
**Grossgasse 6**
**D-6701 Meckenheim(DE)**

(54) **Verfahren zur Herstellung von 5-Cyano-4-nitroimidazolen.**

(57) Verfahren zur Herstellung von 5-Cyano-4-nitroimidazolen der allgemeinen Formel I

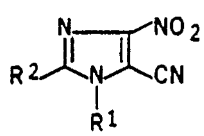

(I),

in der die Reste
$R^1$ Alkyl, Alkoxyalkyl, Cycloalkyl, Aryl, Arylalkyl oder Alkylaryl und
$R^2$ Wasserstoff, Alkyl, Cycloalkyl, Aryl, Arylalkyl oder Alkylaryl,
bedeuten, wobei die genannten organischen Reste noch unter den Reaktionsbedingungen inerte Substituenten tragen können, indem man Dinitroimidazole der allgemeinen Formel II

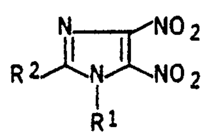

(II)

mit Cyanwasserstoff oder einem Cyanid der allgemeinen Formel III
$M-(CN)_n$ (III),
in der M für ein n-wertiges Metall mit n = 1 bis 3 steht, in einem Lösungs- oder Verdünnungsmittel umsetzt.

EP 0 348 803 A1

## Verfahren zur Herstellung von 5-Cyano-4-nitroimidazolen

Die vorliegende Erfindung betrifft ein neues und verbessertes Verfahren zur Herstellung von 5-Cyano-4-nitroimidazolen.

Aus Helv. Chim. Acta 7, 713-719 (1924) und Rec. Trav. Chim. Pays-Bas 84, 1257 - 1263 (1965) ist bekannt, daß 5-Cyano-4-nitroimidazole der Formel I durch Umsetzung von 5-Chlor-4-nitroimidazolen mit Alkalimetallcyaniden erhalten werden.

Die 5-Chlor-4-nitroimidazole sind nur in schlechter Ausbeute durch Umsetzung von N,N′-Dialkyloxalsäurediamiden mit Phosphorpentachlorid und nachfolgende Nitrierung der gebildeten 5-Chlorimidazole zugänglich (O. Wallach, Liebigs Annalen der Chemie 184, 51 (1877)).

Der Erfindung lag daher die Aufgabe zugrunde, ein neues und verbessertes Verfahren zur Herstellung von 5-Cyano-4-nitroimidazolen der Formel I zu finden und den Nachteilen des bekannten Verfahrens abzuhelfen.

Es wurde nun gefunden, daß man 5-Cyano-4-nitroimidazolen der allgemeinen Formel I

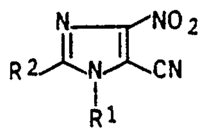

(I),

in der die Reste

$R^1$ Alkyl, Alkoxyalkyl, Cycloalkyl, Aryl, Arylalkyl oder Alkylaryl und

$R^2$ Wasserstoff, Alkyl, Cycloalkyl, Aryl, Arylalkyl oder Alkylaryl,

bedeuten, wobei die genannten organischen Reste noch unter den Reaktionsbedingungen inerte Substituenten tragen können, vorteilhaft erhält, wenn man Dinitroimidazole der allgemeinen Formel II

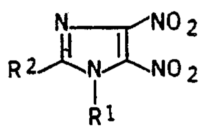

(II)

mit Cyanwasserstoff oder einem Cyanid der allgemeinen Formel III

$M\text{-}(CN)_n$ (III),

in der M für ein n-wertiges Metall mit n = 1 bis 3 steht, in einem Lösungs- oder Verdünnungsmittel umsetzt.

Der Erfolg des erfindungsgemäßen Verfahrens ist überraschend insbesondere im Hinblick darauf, daß selektiv nur die Nitrogruppe in 5-Stellung des Imidazols II ausgetauscht wird.

Die Herstellung von Verbindungen der Formel II ist aus S. S. Novikov et al, Chem. Heterocyclic Compds. 1970, 465 und Chem. Abstr. 73, 66491 z (1970) bekannt. Nach dem dort angegebenen Verfahren ist eine große Zahl von verschieden substituierten Verbindungen der Formel II zugänglich.

So kann der Rest $R^1$ Alkyl, Alkoxyalkyl, Cycloalkyl, Aryl, Arylalkyl oder Alkylaryl bezeichnen. Alkylreste sind z.B. verzweigte oder unverzweigte Reste mit 1 bis 18, insbesondere 1 bis 8 Kohlenstoffatomen wie Methyl, Ethyl, Propyl, Isopropyl, n-Butyl, sek.-Butyl, tert.-Butyl, Pentyl, Hexyl, Heptyl oder Octyl. Alkoxyalkylreste haben z.B. insgesamt 2 bis 8 Kohlenstoffatome wie $C_1$-$C_4$-Alkoxymethyl, Methoxyethyl oder Ethoxyethyl. Cycloalkylreste sind z.B. $C_5$- bis $C_8$-Cycloalkylreste, insbesondere Cyclopentyl- oder Cyclohexyl. Arylreste sind z.B. Phenyl, Naphthyl, ggf. substituiert durch inerte Gruppen wie $C_1$-$C_4$-Alkyl, $C_1$-$C_4$-Alkoxy, Halogen wie Fluor, Chlor oder Brom, Halogenalkyl wie Trifluormethyl, Difluormethyl oder Dichlormethyl oder $C_1$-$C_4$-Alkoxymethyl. Arylalkyl- oder Alkylarylreste sind insbesondere solche mit 7 bis 12 Kohlenstoffatomen wie Tolyl, Xylyl, Phenylethyl oder Benzyl, die ebenso wie die Arylreste substituiert sein können.

Der Rest $R^2$ kann die Bedeutung von $R^1$ haben und darüber hinaus für Wasserstoff stehen. Die organischen Reste $R^1$ und/oder $R^2$ können noch unter den Reaktionsbedingungen inerte Substituenten tragen wie $C_1$-$C_4$-Alkyl- oder Alkoxygruppen oder Halogen wie Fluor, Chlor oder Brom.

Besonders bevorzugte Rest sind für $R^1$ Methyl, Ethyl, Isopropyl oder Benzyl und für $R^2$ Wasserstoff, Methyl, Ethyl, Propyl, Isopropyl, Butyl, Methoxymethyl, Phenyl, 4-Tolyl oder Benzyl.

Nach dem erfindungsgemäßen Verfahren können 4-Nitro-5-cyanoimidazole der Struktur I in einfacher Weise dadurch erhalten werden, daß man Dinitroimidazole II mit Blausäure oder deren Salzen in einem Lösungs- oder Verdünnungsmittel umsetzt.

Als Cyanidquelle dienen Cyanwasserstoff oder Cyanide von ein-, zwei- oder dreiwertigen Metallen, bevorzugt von einwertigen Metallen wie Alkali-metallen, insbesondere Natriumcyanid und Kaliumcyanid.

Als Lösungs- oder Verdünnungsmittel für das erfindungsgemäße Verfahren kommen Alkohole wie Methanol, Ethanol, Propanol, Isopropanol, Butanol, Pentanol, organische Ether wie Tetrahydrofuran oder Dioxan oder Säureamide wie N-Methylpyrrolidon, Dimethylformanid oder N,N,N′N′-Tetramethylharnstoff in Betracht.

Zweckmäßigerweise führt man die Umsetzung zwischen Cyanwasserstoff bzw. Cyanid III und Dinitroimidazol II im Molverhältnis von 0,5 : 1 bis 5 : 1, vorzugsweise 0,9 : 1 bis 2,5 : 1 durch.

In bestimmten Fällen kann es vorteilhaft sein, dem Reaktionsgemisch katalytische Mengen (z.B. 0,0001 - 0,01 Mol pro Mol Dinitroimidazol II) eines Alkali- oder Erdalkalimetallhalogenids, vor allem Bromide und Jodide wie z.B. Natriumbromid, Natriumiodid, Kaliumbromid, Kaliumiodid oder Rubidiumiodid zuzusetzen.

Die Reaktion kann unter milden Bedingungen durchgeführt werden. Temperaturen von 0 bis 150 °C sind im allgemeinen ausreichend. Bevorzugt wird die Umsetzung bei Temperaturen von 20 bis 100 °C vorgenommen.

Die Reaktion wird üblicherweise bei Normaldruck kontinuierlich oder diskontinuierlich in den dafür geeigneten Apparaturen durchgeführt.

Die Isolierung und Reinigung der Produkte erfolgt in an sich bekannter Weise z.B. durch Abfiltrieren der festen Produkte und Umkristallisieren aus geeigneten Lösungsmitteln.

5-Cyano-4-nitroimidazole I sind Zwischenprodukte zur Herstellung von Wirkstoffen, insbesondere von Purinverbindungen (J. Sarasin, E. Wegmann, loc. cit., R.N. Prasad, R.K. Robins, J. Amer. Chem. Soc. **79**, 6401 (1957)).

Die folgenden Beispiele sollen die Erfindung näher erläutern, ohne sie einzuschränken.

## Beispiele

### Beispiel 1: Herstellung von 1-Methyl-4-nitro-5-cyanoimidazol

Eine Lösung von 17,2 g (0,1 mol) 1-Methyl-4,5-dinitroimidazol und 13,0 g (0,2 mol) Kaliumcyanid in 200 ml Methanol wird für 2 Std. auf 40 °C erwärmt. Dann wird das Methanol im Vakuum abgezogen, der Rückstand mit 100 ml Wasser versetzt, das ausgefallene Produkt abgesaugt und getrocknet. Man erhält 10,3 g 1-Methyl-4-nitro-5-cyanoimidazol, Smp. 140 - 141 °C (Lit.: Smp. 141 - 142 °C ), entsprechend einer Ausbeute von 68 %.

### Beispiel 2: Herstellung von 1-Methyl-4-nitro-5-cyanoimidazol

Eine Lösung von 17,2 g (0,1 Mol) 1-Methyl-4,5-dinitroimidazol, 13,0 g (0,2 mol) Kaliumcyanid und 0,08 g (0,0005 mol) Kaliumiodid in 200 ml Methanol wird für 2 Std. auf 40 °C erwärmt. Dann wird wie in Beispiel 1 beschrieben aufgearbeitet. Man erhält 12,0 g 1-Methyl-4-nitro-5-cyanoimidazol, Smp. 140 - 141 °C, entsprechend einer Ausbeute von 79 %.

### Beispiele 3 - 5:

Entsprechend den Beispielen 1 bzw. 2 wurden die folgenden Verbindungen der Struktur I hergestellt.

| Beispiel | Herst. nach Beispiel | $R^1$ | $R^2$ | Ausbeute | Smp (°C ) | Smp. (Lit.) oder Elementaranalyse |
|---|---|---|---|---|---|---|
| 3 | 2 | $-CH_3$ | $-CH_3$ | 71 % | 138 - 139 | Ber: C 43,4; H 3,6 N 33,7 Gef: C 42,9; H 3,8 N 33,8 |
| 4 | 1 | -H | $-CH_2-$ 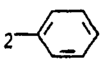 | 60 % | 124 - 125 | 125 - 126°C |
| 5 | 2 | $-CH_3$ | $-CH_2-$ 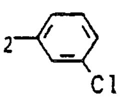 | 62 % | 98 - 100 | Ber: C 52,1; H 3,3 N 20,2; Cl 12,8 Gef: C 52,0; H 3,6 N 19,9; Cl 12,8 |

## Ansprüche

1. Verfahren zur Herstellung von 5-Cyano-4-nitroimidazolen der allgemeinen Formel I

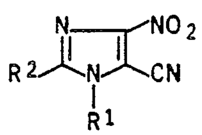

(I),

in der die Reste
$R^1$ Alkyl, Alkoxyalkyl, Cycloalkyl, Aryl, Arylalkyl oder Alkylaryl und
$R^2$ Wasserstoff, Alkyl, Cycloalkyl, Aryl, Arylalkyl oder Alkylaryl, bedeuten, wobei die genannten organischen Reste noch unter den Reaktionsbedingungen inerte Substituenten tragen können, dadurch gekennzeichnet, daß man Dinitroimidazole der allgemeinen Formel II

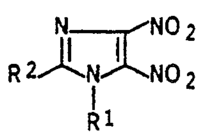

(II)

mit Cyanwasserstoff oder einem Cyanid der allgemeinen Formel III
$M-(CN)_n$ (III),
in der M für ein n-wertiges Metall mit n = 1 bis 3 steht, in einem Lösungs- oder Verdünnungsmittel umsetzt.

2. Verfahren nach Anspruch 1, dadurch gekennzeichnet, daß man die Umsetzung im Molverhältnis Cyanwasserstoff bzw. Cyanid III zu Dinitroimidazol II von 0,5 : 1 bis 5 : 1 vornimmt.

Europäisches Patentamt

# EUROPÄISCHER RECHERCHENBERICHT

Nummer der Anmeldung

EP 89 11 1256

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | HELVETICA CHIMICA ACTA Band 7, 1924, Seiten 713-719, Basel; J. SARASIN et al.: "Synthese de l'heteroxanthine à partir d'un dérivé de I'imidazol" * Seite 716, letzter Absatz - Seite 717, erster Absatz * --- | 1 | C 07 D 233/92 |
| A,D | RECEUIL DES TRAVAUX CHEMIQUES DES PAYS-BAS Band 84, 1965, Seiten 1257-1263, Den Haag, NL; G. E. TROUT et al.: "Studies on imidazoles, Part I: The Action of phosphorus pentachloride on N-Butyl-N'-Ethyloxamide" * Seite 1258, Verbindungen IVa, IVb; Seite 1260 * --- | 1 | |
| A | DE-A-2 243 015 (MERCK & CO. INC.) * Ansprüche 1,9,20 * --- | 1 | |
| A,D | CHEMISTRY OF HETEROCYCLIC COMPOUNDS 1970, Seiten 465-469, New York, USA; S. S. NOVIKOV et al.: "Nitration of imidazoles with various nitrating agents" ----- | 1 | RECHERCHIERTE SACHGEBIETE (Int. Cl.5) C 07 D 233/00 |

Der vorliegende Recherchenbericht wurde für alle Patentansprüche erstellt

| Recherchenort | Abschlußdatum der Recherche | Prüfer |
|---|---|---|
| BERLIN | 12-09-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)